# EUROPEAN PATENT APPLICATION

(11) **EP 2 990 103 A1**
(43) Date of publication of application: **02.03.2016**
(21) Application number: 14787521.5
(22) Date of filing: 09.04.2014
(51) Int. Cl.: B01J 23/10, B01J 29/00, B01J 23/02, B01J 23/16, B01J 23/72, B01J 23/745

(54) **FISCHER-TROPSCH SYNTHESIS CATALYST FOR SYNGAS TO LOW CARBON OLEFINS, MODIFIED MOLECULAR SIEVE CARRIER AND PREPARATION METHOD THEREOF**

(30) Priority: 25.04.2013 CN 201310147608
(71) Applicant: Wuhan Kaidi Engineering Technology Research Institute Co., Ltd., Wuhan, Hubei 430212 (CN)
(72) Inventor: YANG, Weiguang, Wuhan Hubei 430212 (CN); LIU, Qianqian, Wuhan Hubei 430212 (CN); SONG, Dechen, Wuhan Hubei 430212 (CN); LI, Changyuan, Wuhan Hubei 430212 (CN); ZHAN, Xiaodong, Wuhan Hubei 430212 (CN); JIN, Jiaqi, Wuhan Hubei 430212 (CN); ZHANG, Yanfeng, Wuhan Hubei 430212 (CN)
(74) Representative: Zeuner Summerer Stütz
(86) International application number: PCT/CN2014/074974
(87) International publication number: WO 2014/173229

(57) **Abstract**

A Fischer-Tropsch synthesis catalyst for syngas to low carbon olefins, the modified molecular sieve carrier and preparation method thereof. The Fischer-Tropsch catalyst components are molecular sieve carrier and active components, the molecular sieve is Ce salt and/or Pr salt modified Si-Al molecular sieve and/or high-Si molecular sieve carrier. The active component comprises main component of Fe, further Mn and Cu as well as alkaline assistants. The content of modified molecular sieve is 40%-80 wt%. The weight content of Ce salt and/or Pr salt is 1%-20% of that of the molecular sieve. The Ce salt and/or Pr salt modified molecular sieve is prepared by treating Si-Al molecular sieve and/or high-Si molecular sieve with acid solution to obtain H-type molecular sieve, impregnating the H-type molecular sieve in Ce salt solution and/or Pr salt solution, then drying and calcining. The Fischer-Tropsch synthesis catalyst for syngas to low carbon olefins is obtained by impregnating the Ce salt and/or Pr salt modified molecular sieve in the salt solution of active components, then drying and calcining.

## Description

### FIELD OF THE INVENTION

The invention relates to a Fischer-Tropsch synthesis catalyst for syngas to produce light olefins, a modified molecular sieve carrier, and preparation methods thereof, particularly to a catalyst for one-step Fischer-Tropsch synthesis for preparation of light olefin using syngas and a preparation method thereof.

### BACKGROUND OF THE INVENTION

Light olefin is an important basic organic chemical raw material. The light olefin is usually produced by steam cracking the naphtha derived from the crude oil. However, it is urgent to develop substituted raw materials and processes to avoid the supply constraints and the environmental issues. At present, industrialization of the two-step method for preparation of olefin using syngas and methanol has been realized but exists with problems, such as complicate process, long process route, and large investment. The Fischer-Tropsch synthesis for preparation of olefin by one-step reaction is able to well solve the above problems, but the light olefin usually has relatively low selectivity, which restricts the industrialization thereof.

For many years, some research teams have tried to develop high temperature molten iron catalyst for improving the selectivity of the light olefin (FTO) product and have achieved good results. However, the mechanical property of the molten iron catalyst is not good at high temperature, and may result in blockage of the catalyst bed in the fixed bed operation or scale formation on the separating device in the fluid bed process, thereby restricting the application of the molten iron catalyst in the Fischer-Tropsch synthesis for producing the light olefin.

Patent CN1040397C adopts the oxides of Group IIA alkaline earth metals, such as MgO, or the high silica zeolite molecular sieve (or the phosphorus-aluminum zeolite) loaded Fe-Mn catalyst system. Under the effect of the strong base (Group IA metal), such as the K or Cs ion promoters, the catalyst system possesses good selectivity for synthesis of the light olefin. In reaction condition of between 300 and 400°C, the conversion rate of CO is 90%, the light olefin accounts for 66 wt. % of the hydrocarbon products, but CH₄ has relatively high selectivity in the gas-phase products.

Patent CN101219384A adopts the vacuum impregnation method for preparing the catalyst. The primary catalyst component Fe and the promoter are uniformly dispersed on the carrier of the active carbon, thereby realizing the high catalytic activity and good catalytic effect. Under the process conditions of a temperature of between 300 and 400°C, a pressure of between 1 and 2 megapascal, and a space velocity of the syngas of between 400 and 1000 h⁻¹, the conversion rate of CO reaches 95%, the light olefin accounts for 68 wt. % of the gas-phase hydrocarbon, however coking deactivation of the catalyst easily occurs at high temperature.

Patent CN1065026A has disclosed the catalyst for preparing ethylene using syngas and preparation method of the catalyst. More than ten chemical elements, such as Nb, Ga, Pr, Sc, In, Yb, Ce, and La, are added to the catalyst. The selectivity of ethylene reaches 90%, but the conversion rate of CO is relatively low, and the circulation of the syngas increases the costs for the devices and the operation.

De Jong et al. (Krijn P. de Jong et al. Science, 2012, 335,835) have tried to uniformly disperse the iron nanoparticles on weak interactive α-alumina or the carbon nanofibers carrier so that the syngas is directly transformed into C2-C4 light olefin. When the conversion rate of CO is 80%, the light carbon olefin is 50 wt. % of the hydrocarbon products and possesses relatively good coking resistance. However, the preparation process is complicate and difficult for realizing industrialization.

### SUMMARY OF THE INVENTION

In view of the above-described problems, it is one objective of the invention to provide a catalyst for Fischer-Tropsch synthesis for preparation of a light olefin using syngas, a modified molecular sieve, and methods for preparing the same, and particularly methods for preparing a cerium salt and/or a praseodymium salt modified molecular sieve carrier and a catalyst for industrialization application so as to obviously improve the activity and the stability of the catalyst.

Technical scheme of the invention is as follows: a catalyst for Fischer-Tropsch synthesis for preparation of a light olefin using syngas, the catalyst comprises: a molecular sieve carrier and an active component. The molecular sieve carrier is a cerium salt and/or a praseodymium salt modified aluminosilicate molecular sieve carrier and/or a high-silica molecular sieve carrier.

The active component comprises: Fe as a primary component, Mn, Cu, and a basic promoter. The basic promoter is K. The catalyst comprises: between 10 and 35 wt. % of Fe, between 1 and 20 wt. % of Mn, between 1 and 20 wt. % of Cu, between 1 and 10 wt. % of K, and between 40 and 80 wt. % of the modified molecular sieve.

The cerium salt and/or the praseodymium salt account(s) for between 1 and 20 wt. % of the cerium salt and/or the praseodymium salt modified molecular sieve carrier.

The cerium salt and/or the praseodymium salt account(s) for between 10 and 20 wt. % of the cerium salt and/or the praseodymium salt modified molecular sieve carrier.

A method for preparing a cerium salt and/or a praseodymium salt modified molecular sieve carrier, comprises:
1) adding an aluminosilicate molecular sieve SSZ-13 or SAPO-34 and/or a high-silica molecular sieve ZSM-5 to an acid solution of one selected from the group consisting of ammonium sulfate, ammonium nitrate, ammonium chloride, acetic acid, and refluxing a resulting mixture at a temperature of between 26 and 99°C for between 3 and 6 hrs, in which a concentration of the acid solution is between 0.05 and 5 mol/L; filtering a reaction mixture, washing and drying a filter residue, and calcining the filter residue at a temperature of between 400 and 700°C for between 1 and 8 hrs, thereby yielding a hydrogen type molecular sieve;
2) impregnating the hydrogen type molecular sieve obtained in 1) in a cerium salt and/or a praseodymium salt solution having a concentration of between 0.1 and 1 mol/L at a temperature of between 25 and 85°C and a vacuum degree of between 10⁻¹ and 10⁻⁴ pascal for between 15 and 30 hrs; and
3) drying an impregnated molecular sieve obtained in 2) at a temperature of between 70 and 150°C for between 15 and 30 hrs, and calcining a resulting product at a temperature of between 400 and 700°C for between 1 and 8 hrs, thereby yielding the cerium salt and/or the praseodymium salt modified molecular sieve.

Preferably, the cerium salt or the praseodymium salt is a carbonate or a formate thereof.

Preferably, a drying temperature of the modified molecular sieve is between 80 and 130°C, and a drying time of the modified molecular sieve is between 20 and 30 hrs. A calcining temperature of the modified molecular sieve is between 500 and 700°C, and a drying time of the modified molecular sieve is between 4 and 8 hrs.

A method for preparing a catalyst for Fischer-Tropsch synthesis for preparation of a light olefin using syngas, comprises:
1) fully dissolving a ferric salt, a manganese salt, a copper salt, and an alkali or a salt containing a promoter K element according to the following weight percentages: between 10 and 35 wt. % of Fe, between 1 and 20 wt. % of Mn, between 1 and 20 wt. % of Cu, and between 1 and 10 wt. % of K in a definite quantity of an aqueous solvent to yield an aqueous solution, adding a certain weight of a surfactant sodium lauryl sulfate to the aqueous solution while stirring, and continuing stirring to yield a uniform solution; and impregnating a cerium salt and/or the praseodymium salt modified molecular sieve in the uniform solution to yield a mixed solution; and
2) drying the mixed solution at a temperature of between 30 and 70°C for between 1 and 8 hrs, and calcining a dried composition at a temperature of between 400 and 600°C for between 3 and 8 hrs, thereby yielding the catalyst.

Preferably, the ferric salt is ferric nitrate, ferric oxalate, or ferric citrate. The manganese salt, the copper salt, or the salt of the alkali metal promoter is an oxalate, an acetate, or a carbonate thereof.

Preferably, a drying temperature of the mixed solution of 2) is between 50 and 65°C, and a drying time of the mixed solution is between 6 and 8 hrs. A calcining temperature of the mixed solution of 2) is between 500 and 600°C, and a calcining time of the mixed solution is between 6 and 8 hrs.

Preferably, the weight percentages in 1) are as follows: between 10 and 35 wt. % of Fe, between 1 and 10 wt. % of Mn, between 1 and 10 wt. % of Cu, and between 1 and 10 wt. % of K; and the modified molecular sieve accounts for between 60 and 80 wt. % of the catalyst.

Advantages according to embodiments of the invention are summarized as follows:
The modification of the carrier is simple and convenient and decreases the production cost. The catalyst of the invention is prepared by impregnation, which reduces the energy consumption compared to the molten iron catalyst necessitated to be prepared in the high temperature furnace. The preparation process of the invention is simple and easy for industrialization.

The catalytic activity and the stability of the catalyst are obviously improved in the Fischer-Tropsch reaction for synthesizing the olefin. The conversion rate of CO reaches 93%, the selectivity of the light olefin and the ratio of the olefin to the alkane is improved, the light olefin reaches 85 wt. % of the C2-C4 hydrocarbons. Besides, the selectivity of methane is inhibited.

### DETAILED DESCRIPTION OF THE EMBODIMENTS

For further illustrating the invention, experiments detailing a Fischer-Tropsch synthesis catalyst for syngas to light olefins, a modified molecular sieve carrier, and preparation methods thereof are described below. It should be noted that the following examples are intended to describe and not to limit the invention.

A molecular sieve carrier of a catalyst for Fischer-Tropsch synthesis for preparation of a light olefin using syngas is a Ce and/or Pr modified aluminosilicate molecular sieve (SSZ-13, SAPO-34), a high-silica molecular sieve (ZSM-5), or a mixture thereof.

A method for preparing the modified molecular sieve comprises the following steps:
1) adding an aluminosilicate molecular sieve SSZ-13 or SAPO-34 or a high-silica molecular sieve ZSM-5 to an acid solution of one selected from the group consisting of ammonium sulfate, ammonium nitrate, ammonium chloride, acetic acid, and refluxing a resulting mixture at a temperature of between 26 and 99°C for between 3 and 6 hrs, in which a concentration of the acid solution is between 0.05 and 5 mol/L; filtering a reaction mixture, washing and drying a filter residue, and calcining the filter residue at 700°C for 6 hrs to yield a hydrogen type molecular sieve;
2) fully mixing the hydrogen type molecular sieve obtained in 1) with a cerium salt (or a praseodymium salt) solution having a concentration of between 0.1 and 1 mol/L for impregnating the hydrogen type molecular at a temperature of between 25 and 85°C and a vacuum degree of between 10⁻¹ and 10⁻⁴ pascal for between 15 and 30 hrs; and
3) drying an impregnated molecular sieve obtained in 2) at a temperature of between 70 and 150°C for between 15 and 30 hrs, and calcining a resulting product at a temperature of between 400 and 700°C for between 1 and 8 hrs, thereby yielding the cerium salt (or the praseodymium salt) modified molecular sieve.

The cerium salt (or the praseodymium salt) accounts for between 1 and 20 wt. %, and preferably between 10 and 20 wt. %, of the modified molecular sieve.

The cerium salt (or the praseodymium salt) is a nitrate, a formate, a carbonate, or a sulfate thereof; preferably the carbonate or the formate thereof.

The drying temperature of the modified molecular sieve is between 70 and 150°C, and preferably between 80 and 130°C. The drying time of the modified molecular sieve is between 15 and 30 hrs, and preferably between 20 and 30 hrs.

The calcining temperature of the modified molecular sieve is between 400 and 700°C, and preferably between 500 and 700°C. The calcining time of the modified molecular sieve is between 1 and 8 hrs, and preferably between 4 and 8 hrs.

The catalyst for Fischer-Tropsch synthesis comprises: a primary component comprising Fe, an active component comprising Mn and Cu, a basic promoter, and a structure promoter. The basic promoter is K. The structure promoter is selected from the group consisting of Ce (or Pr) modified SSZ-13, SAPO-34, ZSM-5, and a mixture thereof.

The catalyst of the invention can be prepared by the common preparation method in the technical field, and preferably by the impregnation method.

A method for preparing the catalyst of the invention comprises the following steps:
1) preparing the Ce (or Pr) modified molecular sieve;
2) dissolving a ferric salt, a manganese salt, a copper salt, and an alkali or a salt containing a promoter K element according to a certain weight ratio in a definite quantity of an aqueous solvent to yield an aqueous solution, adding a certain weight of a surfactant to the aqueous solution while stirring, and continuing stirring to yield a uniform solution; and impregnating the modified molecular sieve prepared in 1) in the uniform solution at a vacuum degree of between 10⁻¹ and 10⁻⁴ pascal to yield a mixed solution;
3) drying the mixture at a temperature of between 30 and 70°C for between 1 and 8 hrs, and calcining a dried composition at a temperature of between 400 and 600°C for between 3 and 8 hrs to yield the catalyst.

The ferric salt is ferric nitrate, ferric oxalate, ferric citrate, ferric sulfate, etc., and preferably ferric nitrate, ferric oxalate, or ferric citrate.

The manganese salt, the copper salt, or the salt of the alkali metal promoter is an oxalate, an acetate, a citrate, a nitrate, a sulfate, or a carbonate thereof, and preferably the oxalate, the acetate, or the carbonate thereof.

The drying temperature of the mixed solution is between 30 and 70°C, preferably between 50 and 65°C; a drying time of the mixed solution is between 3 and 8 hrs, preferably between 6 and 8 hrs. The calcining temperature of the mixed solution is between 400 and 600°C, preferably between 500 and 600°C; a calcining time of the mixed solution is between 3 and 8 hrs, preferably between 6 and 8 hrs.

The weight percentages of relative components of the catalyst are as follows: between 10 and 35 wt. % of Fe, between 1 and 10 wt. % of Mn, between 1 and 10 wt. % of Cu, between 1 and 10 wt. % of K, and between 40 and 80 wt. % of the modified molecular sieve.

Preferably, the weight percentages in 1) are as follows: between 10 and 35 wt. % of Fe, between 1 and 10 wt. % of Mn, between 1 and 10 wt. % of Cu, and between 1 and 10 wt. % of K; and a preferable ratio of the modified molecular for the benefit of controlling the catalyst is between 60 and 80 wt. %.

A method for using the catalyst in synthesizing the low-carbon olefin using syngas comprises:
adding 1 mL of the catalyst to a constant zone of a fixed bed reactor having an inner diameter of 8 mm; reducing the catalyst by introducing hydrogen at a space velocity of 1500h⁻¹ at 380°C for 8 hrs before reaction; and introducing the syngas having a ratio of hydrogen to carbon of 2 to the reactor at the space velocity of 1000 h⁻¹ at a pressure of 2 megapascal for reaction.

### Example 1

Cerium nitrate and an aluminosilicate molecular sieve SSZ-13 were weighed according to a weight ratio of Ce to the molecular sieve of 1:9. The molecular sieve was added to an ammonium nitrate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a cerium nitrate solution and then impregnated at 85°C at the vacuum degree of 10⁻⁴ pascal for 24 hrs. The molecular sieve after impregnation was dried at 130°C for 20 hrs and calcined at 550°C for 6 hrs so as to obtain a modified molecular sieve.

Ferric oxalate, manganese nitrate, copper citrate, and potassium carbonate were weighed according to a weight ratio of Fe:Mn:Cu:K = 28:5:5:5 and dissolved in water to prepare a solution. A surfactant sodium lauryl sulfate having a weight accounting for 0.1 wt. % of a weight of the solution was added to the solution while stirring. The Ce modified aluminosilicate molecular sieve SSZ-13 was weighed quantitatively according to a weight ratio of Fe to the molecular sieve of 7:15. The solution was added to the molecular sieve under vacuum and uniformly mixed to yield a mixture. The mixture was then dried at 65°C for 4 hrs and calcined at 600°C for 3 hrs to yield a FTO catalyst A in a powder state, and specific components of the catalyst were listed in Table 1. Thereafter, the FTO catalyst A was shaped by pressing, crushed into particles, and sieved. 1 mL of the catalyst having a particle size of between 30 and 60 meshes was added to the fixed bed reactor. Hydrogen was introduced at a space velocity of 1500 h⁻¹ to reduce the catalyst at 380°C for 8 hrs. Then, the syngas (volume ratio of H₂:CO = 2:1) was introduced at the space velocity of 1000 h⁻¹ at 340°C and 2.0 megapascal for continuous reaction. Gas-phase products were detected on line every hour by gas chromatography, and C5+, oxygenates, and CO₂ were excluded from the product selectivity. The reaction results using the catalyst A were shown in Table 2 from which it was known that a conversion rate of CO was 96.1 % and selectivity of the light olefin in light hydrocarbon (excluded from methane) was 90.1 %.

### Example 2

Cerium nitrate and an aluminosilicate molecular sieve SSZ-13 were weighed according to a weight ratio of Ce to the molecular sieve of 1:19. The molecular sieve was added to an ammonium sulfate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a cerium nitrate solution and then impregnated at 25°C at the vacuum degree of 10⁻¹ pascal for 30 hrs. The molecular sieve after impregnation was dried at 150°C for 15 hrs and calcined at 700°C for 6 hrs so as to obtain a modified molecular sieve.

Ferric nitrate, manganese nitrate, copper nitrate, and potassium nitrate were weighed according to a weight ratio of Fe:Mn:Cu:K = 22:6:6:2 and dissolved in water to prepare a solution. A surfactant sodium lauryl sulfate having a weight accounting for 0.1 wt. % of a weight of the solution was added to the solution while stirring. The Ce modified aluminosilicate molecular sieve SSZ-13 was weighed quantitatively according to a weight ratio of Fe to the molecular sieve of 22:65. The solution was added to the molecular sieve under vacuum and uniformly mixed to yield a mixture. The mixture was then dried at 30°C for 8 hrs and calcined at 500°C for 5 hrs to yield a FTO catalyst B in a powder state, and specific components of the catalyst B were listed in Table 1. Evaluation process of the activity of the catalyst was the same as that of Example 1. The reaction results using the catalyst B were shown in Table 2 from which it was known that a conversion rate of CO was 95.6% and selectivity of the light olefin in light hydrocarbon (excluded from methane) was 86.9%.

### Example 3

Cerium nitrate and an aluminosilicate molecular sieve SAPO-34 were weighed according to a weight ratio of Ce to the molecular sieve of 1:99. The molecular sieve was added to an ammonium nitrate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a cerium nitrate solution and then impregnated at 50°C at the vacuum degree of 10⁻³ pascal for 26 hrs. The molecular sieve after impregnation was dried at 70°C for 30 hrs and calcined at 650°C for 2 hrs so as to obtain a modified molecular sieve.

Ferric citrate, manganese oxalate, copper sulfate, and potassium carbonate were weighed according to a weight ratio of Fe:Mn:Cu:K = 18:6:3:1 and dissolved in water to prepare a solution. A surfactant sodium lauryl sulfate having a weight accounting for 0.1 wt. % of a weight of the solution was added to the solution while stirring. The Ce modified aluminosilicate molecular sieve SAPO-34 was weighed quantitatively according to a weight ratio of Fe to the molecular sieve of 1:4. The solution was added to the molecular sieve under vacuum and uniformly mixed to yield a mixture. The mixture was then dried at 40°C for 7 hrs and calcined at 400°C for 8 hrs to yield a FTO catalyst C in a powder state, and specific components of the catalyst C were listed in Table 1. Evaluation process of the activity of the catalyst was the same as that of Example 1. The reaction results using the catalyst C were shown in Table 2 from which it was known that a conversion rate of CO was 94.7% and selectivity of the light olefin in light hydrocarbon (excluded from methane) was 84.7%.

### Example 4

Praseodymium nitrate and an aluminosilicate molecular sieve SAPO-34 were weighed according to a weight ratio of Pr to the molecular sieve of 1:4. The molecular sieve was added to an ammonium sulfate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 60°C at the vacuum degree of 10⁻³ pascal for 22 hrs. The molecular sieve after impregnation was dried at 100°C for 24 hrs and calcined at 500°C for 5 hrs so as to obtain a modified molecular sieve.

Ferric oxalate, manganese sulfate, copper citrate, and potassium carbonate were weighed according to a weight ratio of Fe:Mn:Cu:K = 39:10:9:2 and dissolved in water to prepare a solution. A surfactant sodium lauryl sulfate having a weight accounting for 0.1 wt. % of a weight of the solution was added to the solution while stirring. The Pr modified aluminosilicate molecular sieve SAPO-34 was weighed quantitatively according to a weight ratio of Fe to the molecular sieve of 39:40. The solution was added to the molecular sieve under vacuum and uniformly mixed to yield a mixture. The mixture was then dried at 60°C for 6 hrs and calcined at 550°C for 4 hrs to yield a FTO catalyst D in a powder state, and specific components of the catalyst D were listed in Table 1. Evaluation process of the activity of the catalyst was the same as that of Example 1. The reaction results using the catalyst D were shown in Table 2 from which it was known that a conversion rate of CO was 94.9% and selectivity of the light olefin in light hydrocarbon (excluded from methane) was 88.8%.

### Example 5

Cerium nitrate and a molecular sieve ZSM-5 were weighed according to a weight ratio of Pr to the molecular sieve of 1:9. The molecular sieve was added to an ammonium nitrate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 70°C at the vacuum degree of 10⁻² pascal for 20 hrs. The molecular sieve after impregnation was dried at 120°C for 22 hrs and calcined at 600°C for 3 hrs so as to obtain a modified molecular sieve.

Ferric citrate, manganese nitrate, copper oxalate, and potassium carbonate were weighed according to a weight ratio of Fe:Mn:Cu:K = 11:3:2:1 and dissolved in water to prepare a solution. A surfactant sodium lauryl sulfate having a weight accounting for 0.1 wt. % of a weight of the solution was added to the solution while stirring. The Pr modified molecular sieve ZSM-5 was weighed quantitatively according to a weight ratio of Fe to the molecular sieve of 1:3. The solution was added to the molecular sieve under vacuum and uniformly mixed to yield a mixture. The mixture was then dried at 50°C for 5 hrs and calcined at 600°C for 7 hrs to yield a FTO catalyst E in a powder state, and specific components of the catalyst E were listed in Table 1. Evaluation process of the activity of the catalyst was the same as that of Example 1. The reaction results using the catalyst E were shown in Table 2 from which it was known that a conversion rate of CO was 93.3% and selectivity of the light olefin in light hydrocarbon (excluded from methane) was 87.2%.

### Example 6

Cerium nitrate and a molecular sieve ZSM-5 were weighed according to a weight ratio of Pr to the molecular sieve of 1:19. The molecular sieve was added to an ammonium sulfate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 40°C at the vacuum degree of 10⁻¹ pascal for 28 hrs. The molecular sieve after impregnation was dried at 80°C for 26 hrs and calcined at 700°C for 1 hr so as to obtain a modified molecular sieve.

Ferric sulfate, manganese nitrate, copper citrate, and potassium carbonate were weighed according to a weight ratio of Fe:Mn:Cu:K = 18:6:3:1 and dissolved in water to prepare a solution. A surfactant sodium lauryl sulfate having a weight accounting for 0.1 wt. % of a weight of the solution was added to the solution while stirring. The Pr modified molecular sieve ZSM-5 was weighed quantitatively according to a weight ratio of Fe to the molecular sieve of 1:4. The solution was added to the molecular sieve under vacuum and uniformly mixed to yield a mixture. The mixture was then dried at 70°C for 3 hrs and calcined at 450°C for 6 hrs to yield a FTO catalyst F in a powder state, and specific components of the catalyst F were listed in Table 1. Evaluation process of the activity of the catalyst was the same as that of Example 1. The reaction results using the catalyst F were shown in Table 2 from which it was known that a conversion rate of CO was 94.5% and selectivity of the light olefin in light hydrocarbon (excluded from methane) was 85.6%.

### Example 7

Cerium nitrate, praseodymium nitrate, and an aluminosilicate molecular sieve SSZ-13 were weighed according to a weight ratio of Ce:Pr:molecular sieve = 0.5:0.5:99. The molecular sieve was added to an ammonium nitrate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 85°C at the vacuum degree of 10⁻⁴ pascal for 24 hrs. The molecular sieve after impregnation was dried at 130°C for 20 hrs and calcined at 550°C for 6 hrs so as to obtain a modified molecular sieve.

Ferric oxalate, manganese nitrate, copper citrate, and potassium carbonate were weighed according to a weight ratio of Fe:Mn:Cu:K = 15:10:5:5 and dissolved in water to prepare a solution. A surfactant sodium lauryl sulfate having a weight accounting for 0.1 wt. % of a weight of the solution was added to the solution while stirring. The Ce modified aluminosilicate molecular sieve SSZ-13 was weighed quantitatively according to a weight ratio of Fe to the molecular sieve of 3:13. The solution was added to the molecular sieve under vacuum and uniformly mixed to yield a mixture. The mixture was then dried at 65°C for 4 hrs and calcined at 600°C for 3 hrs to yield a FTO catalyst G in a powder state, and specific components of the catalyst G were listed in Table 1. Evaluation process of the activity of the catalyst was the same as that of Example 1. The reaction results using the catalyst G were shown in Table 2 from which it was known that a conversion rate of CO was 95.2% and selectivity of the light olefin in light hydrocarbon (excluded from methane) was 88.5%.

### Example 8

Cerium nitrate, praseodymium nitrate, and an aluminosilicate molecular sieve SSZ-13 were weighed according to a weight ratio of Ce:Pr:molecular sieve = 1:1:18. The molecular sieve was added to an ammonium sulfate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 25°C at the vacuum degree of 10⁻¹ pascal for 30 hrs. The molecular sieve after impregnation was dried at 150°C for 15 hrs and calcined at 700°C for 6 hrs so as to obtain a modified molecular sieve.

Ferric nitrate, manganese nitrate, copper citrate, and potassium nitrate were weighed according to a weight ratio of Fe:Mn:Cu:K = 35:1:5:4 and dissolved in water to prepare a solution. A surfactant sodium lauryl sulfate having a weight accounting for 0.1 wt. % of a weight of the solution was added to the solution while stirring. The Ce modified aluminosilicate molecular sieve SSZ-13 was weighed quantitatively according to a weight ratio of Fe to the molecular sieve of 7:11. The solution was added to the molecular sieve under vacuum and uniformly mixed to yield a mixture. The mixture was then dried at 30°C for 8 hrs and calcined at 500°C for 5 hrs to yield a FTO catalyst H in a powder state, and specific components of the catalyst H were listed in Table 1. Evaluation process of the activity of the catalyst was the same as that of Example 1. The reaction results using the catalyst H were shown in Table 2 from which it was known that a conversion rate of CO was 96.3% and selectivity of the light olefin in light hydrocarbon (excluded from methane) was 89.3%.

### Example 9

Cerium nitrate, praseodymium nitrate, and an aluminosilicate molecular sieve SAPO-34 were weighed according to a weight ratio of Ce:Pr:molecular sieve = 1:1:8. The molecular sieve was added to an ammonium nitrate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 50°C at the vacuum degree of 10⁻³ pascal for 26 hrs. The molecular sieve after impregnation was dried at 70°C for 30 hrs and calcined at 650°C for 2 hrs so as to obtain a modified molecular sieve.

Ferric citrate, manganese oxalate, copper sulfate, and potassium carbonate were weighed according to a weight ratio of Fe:Mn:Cu:K = 20:10:5:2 and dissolved in water to prepare a solution. A surfactant sodium lauryl sulfate having a weight accounting for 0.1 wt. % of a weight of the solution was added to the solution while stirring. The Ce modified aluminosilicate molecular sieve SAPO-34 was weighed quantitatively according to a weight ratio of Fe to the molecular sieve of 20:63. The solution was added to the molecular sieve under vacuum and uniformly mixed to yield a mixture. The mixture was then dried at 40°C for 7 hrs and calcined at 400°C for 8 hrs to yield a FTO catalyst I in a powder state, and specific components of the catalyst I were listed in Table **1.** Evaluation process of the activity of the catalyst was the same as that of Example **1.** The reaction results using the catalyst I were shown in Table 2 from which it was known that a conversion rate of CO was 96.3% and selectivity of the light olefin in light hydrocarbon (excluded from methane) was 87.6%.

### Example 10

Cerium nitrate and an aluminosilicate molecular sieve SAPO-34 were weighed according to a weight ratio of Ce to the molecular sieve of 1:4. The molecular sieve was added to an ammonium sulfate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 60°C at the vacuum degree of 10⁻³ pascal for 22 hrs. The molecular sieve after impregnation was dried at 100°C for 24 hrs and calcined at 500°C for 5 hrs so as to obtain a modified molecular sieve.

Ferric oxalate, manganese sulfate, copper citrate, and potassium carbonate were weighed according to a weight ratio of Fe:Mn:Cu:K = 10:5:3:2 and dissolved in water to prepare a solution. A surfactant sodium lauryl sulfate having a weight accounting for 0.1 wt. % of a weight of the solution was added to the solution while stirring. The Pr modified aluminosilicate molecular sieve SAPO-34 was weighed quantitatively according to a weight ratio of Fe to the molecular sieve of 1:8. The solution was added to the molecular sieve under vacuum and uniformly mixed to yield a mixture. The mixture was then dried at 60°C for 6 hrs and calcined at 550°C for 4 hrs to yield a FTO catalyst J in a powder state, and specific components of the catalyst J were listed in Table 1. Evaluation process of the activity of the catalyst was the same as that of Example 1. The reaction results using the catalyst J were shown in Table 2 from which it was known that a conversion rate of CO was 95% and selectivity of the light olefin in light hydrocarbon (excluded from methane) was 85.8%.

### Example 11

Cerium nitrate and a molecular sieve ZSM-5 were weighed according to a weight ratio of Pr to the molecular sieve of 1:99. The molecular sieve was added to an ammonium nitrate solution having a concentration of 0.1 mol/L and a resulting mixture was refluxed at 80°C for 5 hrs and then filtered. A filter residue was washed, dried, and calcined at 700°C for 6 hrs to yield a hydrogen type molecular sieve. Thereafter, the hydrogen type molecular sieve was uniformly mixed with a praseodymium nitrate solution and then impregnated at 70°C at the vacuum degree of 10⁻² pascal for 20 hrs. The molecular sieve after impregnation was dried at 120°C for 22 hrs and calcined at 600°C for 3 hrs so as to obtain a modified molecular sieve.

Ferric citrate, manganese nitrate, copper oxalate, and potassium carbonate were weighed according to a weight ratio of Fe:Mn:Cu:K = 23:1:9:7 and dissolved in water to prepare a solution. A surfactant sodium lauryl sulfate having a weight accounting for 0.1 wt. % of a weight of the solution was added to the solution while stirring. The Pr modified molecular sieve ZSM-5 was weighed quantitatively according to a weight ratio of Fe to the molecular sieve of 23:60. The solution was added to the molecular sieve under vacuum and uniformly mixed to yield a mixture. The mixture was then dried at 50°C for 5 hrs and calcined at 600°C for 7 hrs to yield a FTO catalyst K in a powder state, and specific components of the catalyst K were listed in Table 1. Evaluation process of the activity of the catalyst was the same as that of Example 1. The reaction results using the catalyst K were shown in Table 2 from which it was known that a conversion rate of CO was 94.2% and selectivity of the light olefin in light hydrocarbon (excluded from methane) was 86.3%.

**Table 1 Specific components of catalysts A-K**

| Catalyst | Compositions of modified carrier by weight percentage | Compositions of catalyst by weight percentage |
|---|---|---|
| A | 10% Ce | 28 Fe:5 Mn:5 Cu:5 K:60 modified molecular sieve |
| B | 5% Ce | 22 Fe:6 Mn:5 Cu:2 K:65 modified molecular sieve |
| C | 1% Ce | 18 Fe:6 Mn:3 Cu:1 K:72 modified molecular sieve |
| D | 20% Pr | 39 Fe:10 Mn:9 Cu:2 K:40 modified molecular sieve |
| E | 10% Pr | 22 Fe:6 Mn:4 Cu:2 K:66 modified molecular sieve |
| F | 5% Pr | 18 Fe:6 Mn:3 Cu:1 K:72 modified molecular sieve |
| G | 0.5% Ce + 0.5% Pr | 15 Fe:10 Mn:5 Cu:5 K:65 modified molecular sieve |
| H | 5% Ce + 5% Pr | 35 Fe:1 Mn:5 Cu:4 K:55 modified molecular sieve |
| I | 10% Ce + 10% Pr | 20 Fe:10 Mn:5 Cu:2 K:63 modified molecular sieve |
| J | 20% Ce | 10 Fe:5 Mn:3 Cu:2 K:80 modified molecular sieve |
| K | 1% Pr | 23 Fe: 1 Mn:9 Cu:7 K:60 modified molecular sieve |

**Table 2 Evaluation results of catalyst activity**

| Catalyst | Selectivity (%) | | | | |
|---|---|---|---|---|---|
| | Conversion rate of CO (%) | CH₄ | C2-C4 alkane | C2=-C4= olefin | Ratio of light olefin to C2-C4 hydrocarbon |
| A | 96.1 | 10.7 | 8.2 | 74.5 | 90.1 |
| B | 95.6 | 13.1 | 10.5 | 69.8 | 86.9 |
| C | 94.7 | 14.5 | 12.2 | 67.4 | 84.7 |
| D | 94.9 | 12.5 | 9.0 | 71.6 | 88.8 |
| E | 93.3 | 11.7 | 10.1 | 68.5 | 87.2 |
| F | 94.5 | 13.6 | 11.3 | 68.7 | 85.6 |
| G | 95.2 | 13.5 | 9.5 | 73.2 | 88.5 |
| H | 96.3 | 11.1 | 8.6 | 71.5 | 89.3 |
| I | 96.3 | 14.6 | 10.0 | 70.5 | 87.6 |
| J | 95 | 12.5 | 11.5 | 69.4 | 85.8 |
| K | 94.2 | 14.8 | 11.4 | 72.1 | 86.3 |

## Claims

1. A catalyst for Fischer-Tropsch synthesis for preparation of a light olefin using syngas, the catalyst comprising a molecular sieve carrier and an active component; **characterized in that** the molecular sieve carrier is a cerium salt and/or a praseodymium salt modified aluminosilicate molecular sieve carrier and/or a high-silica molecular sieve carrier.

2. The catalyst of claim 1, **characterized in that** the active component comprises: Fe as a primary component, Mn, Cu, and a basic promoter; the basic promoter is K; and the catalyst comprises: between 10 and 35 wt. % of Fe, between 1 and 20 wt. % of Mn, between 1 and 20 wt. % of Cu, between 1 and 10 wt. % of K, and between 40 and 80 wt. % of the modified molecular sieve.

3. The catalyst of claim 1 or 2, **characterized in that** the cerium salt and/or the praseodymium salt account(s) for between 1 and 20 wt. % of the cerium salt and/or the praseodymium salt modified molecular sieve carrier.

4. The catalyst of claim 1 or 2, **characterized in that** the cerium salt and/or the praseodymium salt account(s) for between 10 and 20 wt. % of the cerium salt and/or the praseodymium salt modified molecular sieve carrier.

5. A method for preparing a cerium salt and/or a praseodymium salt modified molecular sieve carrier, comprising:
1) adding an aluminosilicate molecular sieve SSZ-13 or SAPO-34 and/or a high-silica molecular sieve ZSM-5 to an acid solution of one selected from the group consisting of ammonium sulfate, ammonium nitrate, ammonium chloride, acetic acid, and refluxing a resulting mixture at a temperature of between 26 and 99°C for between 3 and 6 hrs, in which a concentration of the acid solution is between 0.05 and 5 mol/L; filtering a reaction mixture, washing and drying a filter residue, and calcining the filter residue at a temperature of between 400 and 700°C for between 1 and 8 hrs, thereby yielding a hydrogen type molecular sieve;
2) impregnating the hydrogen type molecular sieve obtained in 1) in a cerium salt and/or a praseodymium salt solution having a concentration of between 0.1 and 1 mol/L at a temperature of between 25 and 85°C and a vacuum degree of between 10⁻¹ and 10⁻⁴ pascal for between 15 and 30 hrs; and
3) drying an impregnated molecular sieve obtained in 2) at a temperature of between 70 and 150°C for between 15 and 30 hrs, and calcining a resulting product at a temperature of between 400 and 700°C for between 1 and 8 hrs, thereby yielding the cerium salt and/or the praseodymium salt modified molecular sieve.

6. The method of claim 5, **characterized in that** the cerium salt or the praseodymium salt is a carbonate or a formate thereof.

7. The method of claim 5 or 6, **characterized in that**
a drying temperature of the modified molecular sieve is between 80 and 130°C, and a drying time of the modified molecular sieve is between 20 and 30 hrs; and
a calcining temperature of the modified molecular sieve is between 500 and 700°C, and a calcining time of the modified molecular sieve is between 4 and 8 hrs.

8. A method for preparing a catalyst for Fischer-Tropsch synthesis for preparation of a light olefin using syngas, comprising:
1) fully dissolving a ferric salt, a manganese salt, a copper salt, and an alkali or a salt containing a promoter K element according to the following weight percentages: between 10 and 35 wt. % of Fe, between 1 and 20 wt. % of Mn, between 1 and 20 wt. % of Cu, and between 1 and 10 wt. % of K in a definite quantity of an aqueous solvent to yield an aqueous solution, adding a certain weight of a surfactant sodium lauryl sulfate to the aqueous solution while stirring, and continuing stirring to yield a uniform solution; and impregnating a cerium salt and/or the praseodymium salt modified molecular sieve in the uniform solution to yield a mixed solution; and
2) drying the mixed solution at a temperature of between 30 and 70°C for between 1 and 8 hrs, and calcining a dried composition at a temperature of between 400 and 600°C for between 3 and 8 hrs, thereby yielding the catalyst.

9. The method of claim 8, **characterized in that** the ferric salt is ferric nitrate, ferric oxalate, or ferric citrate; and the manganese salt, the copper salt, or the salt of the alkali metal promoter is an oxalate, an acetate, or a carbonate thereof.

10. The method of claim 8 or 9, **characterized in that**
a drying temperature of the mixed solution of 2) is between 50 and 65oC, and a drying time of the mixed solution is between 6 and 8 hrs; and
a calcining temperature of the mixed solution of 2) is between 500 and 600°C, and a calcining time of the mixed solution is between 6 and 8 hrs.

11. The method of claim 8 or 9, **characterized in that** the weight percentages in 1) are as follows: between 10 and 35 wt. % of Fe, between 1 and 10 wt. % of Mn, between 1 and 10 wt. % of Cu, and between 1 and 10 wt. % of K; and the modified molecular sieve accounts for between 60 and 80 wt. % of the catalyst.
